# EUROPEAN PATENT SPECIFICATION

(11) **EP 2 775 857 B1**
(45) Date of publication and mention of the grant of the patent: **01.01.2020**
(21) Application number: 12810110.2
(22) Date of filing: 09.11.2012
(51) Int. Cl.: A23K 10/30, A23K 10/33, A23K 20/189, A23K 40/10, A23K 40/20, A23K 50/30, A23K 50/75, A23K 50/80, C12N 9/24

(54) **A FEED COMPOSITION SUPPLEMENTED WITH A XYLANASE**
MIT XYLANASE ERGÄNZTE FUTTERZUSAMMENSETZUNG
COMPOSITION ALIMENTAIRE POUR ANIMAUX CONTENANT UNE XYLANASE

(30) Priority: 09.11.2011 EP 11188480
(43) Date of publication of application: 17.09.2014
(73) Proprietor: Puratos N.V., 1702 Groot-Bijgaarden (BE)
(72) Inventor: BRUYER, Denis, B-1370 JODOIGNE (BE); GEORIS, Jacques, B-4000 LIEGE (BE); DORGEO, Valérie, B-6880 BERTRIX (BE)
(74) Representative: De Clercq, Ann G. Y.
(86) International application number: PCT/EP2012/072307
(87) International publication number: WO 2013/068550

(56) References cited:
- WO-A2-01/42433
- WO-A2-2010/083518
- DE-A1- 19 531 944
- US-A- 5 902 581
- US-B1- 7 060 482
- WU ET AL: "Overexpression of GH10 endoxylanase XynB from Thermotoga maritima in Escherichia coli by a novel vector with potential for industrial application", ENZYME AND MICROBIAL TECHNOLOGY, STONEHAM, MA, US, vol. 42, no. 3, 15 January 2008 (2008-01-15), pages 230-234, XP022422534, ISSN: 0141-0229, DOI: 10.1016/J.ENZMICTEC.2007.09.013

## Description

### Field of the invention

The present invention relates to an animal feed supplemented with an hyperthermostable and hyperthermophilic xylanase.

### Background of the invention and state of the art

Xylanases have been used as feed additives for several years. Indeed some but not all xylanases have been shown to improve one or both of the animal absolute body weight gain (BWG) and the Feed Conversion Ratio (FCR) of a given feed.

Xylanases used in feed may for example allow nutritionists to reduce the energy requirements in the diets without affecting the zootechnical performances of animals.

Xylanases increase the metabolizable or net energy of the raw ingredients and therefore increase the total metabolizable or net energy content of the diets. Zootechnical performances can therefore be maintained with less raw energy in less expensive diets (less fat/oil; more fiber).

A possible benefit linked to the use of xylanase is a better release of (micro)nutrients entrapped within the cell walls of the feed. Such entrapment is due to the presence of non-starch polysaccharides that are resistant to the digestion by the animal.

In order to be effective, xylanases that are used as feed additives should be both stable and active at a pH and a temperature close to the conditions found in the gastrointestinal tract of the animal.

WO 95/29997 describes thermostable xylanases and mentions their incorporation into animal feed. The thermostability is defined as resistance to one minute at 95°C, coupled to the capacity to subsequently hydrolyse a solution of wheat arabinoxylan at 40°C. However, after a five minutes heat treatment, the disclosed xylanases significantly lose their activity. DE 195 31 994 describes thermostable endo-xylanases A and B derived from *Thermotoga neapolitana* and mentions that these endo-xylanases can be added to animal feed. US 5 902 581 A discloses xylanase obtained from *Acidothermus cellulolyticus* with an acidic pH optimum. WO 2010/083518 discloses enzymes TH1 and TH4 having xylanase activity.
Several species and strains of *Thermotoga* have been described to produce one or more hyperthermophilic and thermostable endoxylanase(s). *Thermotoga maritima* produces two thermostable endoxylanases, designated XynA and XynB. XynA and XynB occur as proteins with apparent molecular masses of about 120 and 40 kDa. Maximum activity at the optimal pH (pH 6.2 and pH 5.4 for XynA and XynB, respectively) is measured at about 92°C for XynA and at about 105°C for XynB (Winterhalter and Liebl, 1995, Appl. Env. Microbiol., 61, 1810-1815).

### Summary of the invention

A first aspect of the present invention is an animal feed being selected from the group consisting of silage, pelletized feed and mash feed, said animal feed being supplemented with a composition comprising a xylanase, wherein said xylanase is hyperthermophilic and hyperthermostable, wherein
the optimum temperature of the xylanolytic activity present in this said composition is higher than 80°C, more preferably higher than 85°C, still more preferably higher than 90°C, wherein
the ratio of activity of the xylanolytic activity present in this composition at optimal temperature and at 40°C is higher than 10, more preferably higher than 20, and wherein
more than 70% of the xylanolytic activity present in this composition is resistant to 30 minutes of heating at 90°C.

More preferably, the hyperthermophilic and hyperthermostable xylanase has more than 80% of identity and/or more than 90% of similarity with a xylanase selected from the group consisting of SEQ.ID.NO:1, SEQ.ID.NO:2, SEQ.ID.NO:3, SEQ.ID.NO:4, SEQ.ID.NO:5, amino acids 21-332 of SEQ.ID.NO:1, of SEQ.ID.NO:2, of SEQ.ID.NO:3, of SEQ.ID.NO:4 and of SEQ.ID.NO:5 and possibly this animal feed comprises between 0.08 and 40 mg/kg of the said hyperthermophilic and hyperthermostable (pure) xylanase.

Possibly, this animal feed is liquid and preferably comprises between 0.08 and 40 mg/l of the hyperthermophilic and hyperthermostable (pure) xylanase.

Preferably, the animal feed comprises at least 50% (on dry weight) of plant material, which is more preferably selected from the group consisting of cereals, legumes, beet molasse, potato pulps and peanut meal.

Preferably, the animal feed comprises at least 5% (dry weight) proteins and/or at least 2% (dry weight) fat.

A related aspect is an animal feed comprising between 0.08 and 40 mg/kg (on dry weight) of a polypeptide having more than 80% of identity and/or more than 90% of similarity with a polypeptide selected from the group consisting of SEQ.ID.NO:1, SEQ.ID.NO:2, SEQ.ID.NO:3, SEQ.ID.NO:4, SEQ.ID.NO:5, amino acids 21-332 of SEQ.ID.NO:1, of SEQ.ID.NO:2, of SEQ.ID.NO:3, of SEQ.ID.NO:4 and of SEQ.ID.NO:5.

Another related aspect is the (non therapeutical) use of the animal feed for improving the body weight gain and/or the feed conversion ratio in an animal being preferably a non-ruminant vertebrate or a crustacean and more preferably a fish, pig or poultry. Another related aspect is the (non therapeutical) use of a hyperthermophilic and hyperthermostable xylanase for improving the body weight gain and/or the feed conversion ratio in an animal preferably a non-ruminant vertebrate or a crustacean and more preferably a fish, pig or poultry, wherein said hyperthermophilic and hyperthermostable xylanase has a temperature optimum higher than 80°C, wherein more than 70% of the xylanolytic activity of said hyperthermophilic and hyperthermostable xylanase is resistant to 30 minutes of heating at 90°C and wherein the ratio of activity at optimal temperature and at 40°C of said hyperthermophilic and hyperthermostable xylanase is higher than 10.

Still another related aspect is a method to produce the animal feed comprising the steps of
a) selecting a feed comprising hemicellulose;
b) adding a composition comprising hyperthermophilic and hyperthermostable xylanase, wherein
more than 70% of the xylanolytic activity present in the composition added at step b) of this method is resistant to 30 minutes of heating the said composition at 90°C and the optimum temperature of the xylanolytic activity present in this composition is higher than 80°C (preferably higher than 85°C, more preferably higher than 90°C), and wherein the ratio of activity of the xylanolytic activity present in said composition at optimal temperature and at 40°C is higher than 10. Preferably, the xylanase activity present in said composition is measured using 3% (w:v) xylan, more preferably using 3% birchwood xylan, as substrate and/or the xylanase activity present in said composition is measured after a 15-minute reaction.

### Detailed description of the invention

The inventors have found that the addition of an hyperthermophilic (and hyperthermostable) xylanase to a feed resulted into an improved feed conversion ratio by animals.

Although the optimal working temperature of the hyperthermophilic (endo)xylanases is much higher than the temperature of the intestinal tract of the animal, the inventors have found that these enzymes improved the Body Weight Gain and/or Feed Conversion Ratio, even when compared to commonly used feed (endo)xylanases. More particularly the inventors have even found that xylanases that are both hyperthermophilic (and hyperthermostable) show better results than thermophilic (and thermostable) xylanases.

Both the animal absolute body weight gain (BWG) and the Feed Conversion Ratio (FCR) were improved, in the case of animals fed with mash feed supplemented with hyperthermophilic and hyperthermostable xylanase, by comparison to animals fed with non-supplemented mash feed and to animals wherein the mash feed has been supplemented with a xylanase mostly active at 37-50°C.

The FCR refers to the ratio between the amounts of feed consumed by an animal relative to its weight gain. A lower FCR ratio is indicative of a more efficient utilization of the feed.

These results contrast, in the case of the hyperthermophilic and hyperthermostable xylanase used, with the extremely low xylanase activity measured at 37°C.

A first aspect of the present invention is a feed (an animal feed) selected from the group consisting of silage, pelletized feed and mash feed, the said animal feed being supplemented with (and/or comprising) a composition comprising an hyperthermophilic (i.e. the temperature optimum of the (overall) xylanolytic activity is above 80°C, preferably above 90°C or 95°C and the ratio of activity at optimal temperature and at 40°C is above 10 or even above 15 or 20) and hyperthermostable xylanase (i.e. the (overall) xylanolytic activity of this composition is very resistant to heat inactivation (denaturation), wherein more than 70% of the activity resists to 30 minutes of heating at 90°C the additive containing the xylanase in an oil bath).

The feed may comprise between 0.08 mg/kg feed and 40 mg/kg feed of the hyperthermophilic and hyperthermostable xylanase, more preferably between 0.2 mg/kg feed and 20 mg/kg feed, still more preferably between 0.4 mg/kg feed and 16 mg/kg feed of the (pure) enzyme.

Preferably, the feed comprises between 10 DXU/kg feed and 5000 DXU/kg feed of the hyperthermophilic and hyperthermostable xylanase, more preferably between 25 DXU/kg feed and 2500 DXU/kg feed, still more preferably between 50 DXU/kg feed and 2000 DXU/kg feed.

In the context of the present invention, one unit of the xylanases (DXU) is defined as the amount of enzyme needed to release 1 pmole of reducing sugar (expressed as xylose) per minute from a solution of 3% birchwood xylan at pH 6 and at 70°C, unless other values are explicitly mentioned.

Also disclosed herein is a feed additive (an animal feed additive) comprising a hyperthermophilic and hyperthermostable xylanase as defined elsewhere herein.

In the context of the present invention, "hyperthermostable xylanase" refers to a xylanase wherein more than 70% of the (overall) xylanolytic activity (present in this composition) is resistant to (able to resist to and/or able to be maintained after) 30 minutes of heating (this composition) at 90°C. The term "overall xylanolytic activity" refers to the xylanolytic activity of the hyperthermophilic and hyperthermostable xylanase, and also to the activity of other (contaminant) xylanases that are less preferably present in the composition, added to the animal feed, as disclosed in the present invention.

Preferably, at least 75%, 80%, 85%, 90%, 91%, 92%, 93%, 94%, 95%, 96%, 97%, 98%, 99% or even up to 100% of the (overall) xylanolytic activity present in this feed and/or in this feed additive is able to be maintained after 30 minutes (or 45 minutes or 1 hour) of heating (this feed and/or this feed additive) at 90°C (or at 95°C).

The optimal temperature (temperature optimum) for the (overall) xylanolytic activity present in the composition added to the feed of the present invention is higher than 80°C, more preferably higher than 85°C, 90°C or even higher than 95°C.

The ratio of activity of the (overall) xylanolytic activity present in the composition added to the feed of the present invention (comprising the hyperthermophilic and hyperthermostable xylanase) at optimal temperature (e.g. 80°C, 85°C, 90°C, 95°C or even about 100°C) and at 40°C (or 37°C) is higher than 10, more preferably higher than 20.

In the context of the present invention the term "enzyme with xylanolytic activity", "endoxylanase" or "xylanase" refers to an enzyme (for instance a recombinant, at least partially purified xylanase or, less preferably to a mixture of enzymes) that is (are) able to hydrolyze internal glycosyl bonds linking xylose residues in xylose-containing polysaccharides. Such glycosyl bonds can be for instance the beta-1,4-glycosyl bond in beta-D-xylopyranosyl-1,4-beta-D-xylopyranosyl units of such polysaccharides.

The preferred enzymes with xylanolytic activity are endoxylanases (EC 3.2.1.8.).

The preferred enzymes with xylanolytic activity are endoxylanases from glycoside hydrolases family 10.

Among the most preferred enzymes with xylanolytic activity (hyperthermophilic and hyperthermostable xylanase) are enzymes derived from a strain of *Thermotoga maritima* and is still more preferably XynB (SEQ.ID.NO:1) or close variants thereof (e.g. SEQ.ID.NO:2-5), or comprise the most conserved region of the same sequences (SEQ.ID.NOs:1-5), preferably wherein this most conserved region encompasses the glycoside hydrolases Family 10 xylanase motif (a conserved part of the glycoside hydrolases Family 10 xylanase motif spans over regions 21-332 of SEQ.ID.NO:1-5, or from amino acid 21 to the last amino acid 344, 346 or 347 of these SEQ.ID.NOs:1-5) or share a significant identity (or similarity) with these SEQ.ID.NO.1-5 or regions 21-332 (or 21 to the last amino acid 344, 346 or 347) of these SEQ.ID.NOs:1-5. The added enzyme(s) with xylanolytic activity (hyperthermophilic and hyperthermostable xylanase) may also be a fusion protein comprising SEQ.ID.NOs:1-5 or a fusion protein comprising a peptide sharing a significant identity (or similarity) with these SEQ.ID.NO:1-5 or with regions 21-332 of these SEQ.ID.NOs: 1-5.

"Significant identity" in the context of the present invention refers to at least 75% identity, preferably at least 80%, more preferably at least 85%, 90%, 91%, 92%, 93%, 94%, 95%, 96%, 97%, 98% or even at least 99%. Preferably, the identity is measured over the full length of the (recited) sequence (or fragment), such as over the full length of SEQ.ID.NOs:1-5 or over the full length of peptide consisting of amino acids 21-332 (or of amino acids 21 to the last amino acid) of SEQ.ID.NOs:1-5. "Significant similarity" in the context of the present invention refers to at least 85% similarity, preferably at least 90%, more preferably at least 91%, 92%, 93%, 94%, 95%, 96%, 97%, 98% or even at least 99%. Preferably, the similarity is measured over the full length of the (recited) sequence (or fragment), such as over the full length of SEQ.ID.NOs:1-5 or over the full length of peptide consisting of amino acids 21-332 (or of amino acids 21 to the last amino acid) of SEQ.ID.NOs:1-5.

The relatedness between two amino acid sequences or between two nucleotide sequences is described by the parameter "identity" or "similarity". For purposes of the present invention, the degree of similarity (and of identity) between two amino acid sequences is determined as in WO 2010/0142 697 using the Needleman-Wunsch algorithm as implemented in the Needle program of the EMBOSS package, preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues x 100)/(Length of Alignment - Total Number of Gaps in Alignment). The output of Needle labeled "longest similarity" (obtained using the -nobrief option) is used as the percent similarity and is calculated as follows: (Similar Residues x 100)/(Length of Alignment - Total Number of Gaps in Alignment)
Xylanases (and/or the composition comprising the hyperthermophilic and hyperthermostable xylanase) are advantageously added during the preparation of the feed, being a solid preparation such as mash or pellet or, being a liquid.

The pelleting process usually includes steps at high temperature such as steam injection.

Conversely, animal feed can be produced without the use of heating steps (e.g. beyond 50°C), for instance for feed that are not pelleted.

A related aspect is a (an animal) feed (and/or a (feed) premix) comprising per kg between 0.08 mg and 400 mg (preferably between 0.2 mg/kg and 40 mg/kg, more preferably between 0.4 mg/kg and 20 mg/kg) of a polypeptide having more than 75% (preferably more than 80%, 85%, 90%, 91%, 92%, 93%, 94%, 95%, 96%, 97%, 98%, 99% or even 100%) identity (or similarity) with a polypeptide selected from the group consisting of SEQ.ID.NO:1, SEQ.ID.NO:2, SEQ.ID.NO:3, SEQ.ID.NO:4, SEQ.ID.NO:5, amino acids 21-332 (or from amino acid 21 to the last amino acid 344, 346 or 347) of SEQ.ID.NO:1, of SEQ.ID.NO:2, of SEQ.ID.NO:3, of SEQ.ID.NO:4 and of SEQ.ID.NO:5, said polypeptide being hyperthermostable and having hyperthermophilic xylanase activity.

Preferably, the (animal) feed of the present invention comprises at least 50% (on dry weight) of plant material, most preferably at least 55% or even at least 60%.

Preferably, this plant material is selected from the group consisting of cereals, legumes, beet molasse, potato pulps and peanut meal and can also include mixture thereof such as cereals and legumes.

Preferably (or in addition), the feed of the present invention (comprising at least 50% of plant material) comprises at least 5% (dry weight) proteins, more preferably at least 10%, at least 15% or even at least 20% proteins.

Preferably (or in addition), the feed of the present invention (comprising at least 50% of plant material and/or at least 5% of proteins) comprises at least 2% (dry weight) fat, more preferably at least 5% or even at least 8% of fat.

The feed of the present invention is selected from the group consisting of silage, pelletized feed and mash feed.

Another related aspect of the present invention is the (non-therapeutic) use of the animal feed of the present invention (and/or the use of a (feed) premix and/or the use of a feed additive) and/or the use of the hyperthermophilic and hyperthermostable xylanase of the present invention for improving the body weight gain and/or the feed conversion ratio of a (growing) animal (and most preferably not for producing excessive pathologic weight gain).

The preferred hyperthermophilic and hyperthermostable xylanase for (non-therapeutically) improving the body weight gain (BWG) and/or the feed conversion ratio (FCR) of a (growing) animal have more than 70%, 75%, 80%, 85% 90%, 91%, 92%, 93%, 94%, 95%, 96%, 97%, 98% or even more than 99% of the (overall) xylanolytic activity that is resistant to 1 hour of heating at 90°C (or 95°C) .

Alternatively, or in addition, the preferred hyperthermophilic and hyperthermostable xylanase used for (non-therapeutically) improving the body weight gain (BWG) and/or the feed conversion ratio (FCR) of a (growing) animal are from glycoside hydrolases Family 10.

The most preferred hyperthermophilic and hyperthermostable xylanases used for (non-therapeutically) improving the body weight gain (BWG) and/or the feed conversion ratio (FCR) of a (growing) animal are from *Thermotoga,* and still more preferably share a significant homology (similarity) with SEQ.ID.NOs 1-5, or with fragments 21-332 of SEQ.ID.NOs 1-5, or comprise (as fusion protein) a peptide sharing a significant homology (similarity) with fragments 21-332 of SEQ.ID.NOs 1-5.

In the context of the present invention, "animals" refers preferably to non-human animals and/or are animals having no (apparent) pathologies and/or no pathologies related to a weight deficit.

The animals of the present invention are preferably non-ruminant animals and/or are mono-gastric animals.

More preferably, the animals are selected from the group consisting of pigs, poultry, fish and crustaceans, still more preferably are pigs and/or poultry.

### Origin and preparation of the enzyme

Several species and strains of *Thermotoga* has been described to produce one or more hyperthermophilic and thermostable endoxylanase(s). *Thermotoga maritima* produces two thermostable endoxylanases, designated XynA and XynB. XynA and XynB occur as proteins with apparent molecular masses of about 120 and 40 kDa. Maximum activity at the optimal pH (pH 6.2 and pH 5.4 for XynA and XynB, respectively) has been measured at about 92°C for XynA and at about 105°C for Xyn B (Winterhalter and Liebl, 1995, Appl. Env. Microbiol., 61, 1810-1815). WO93/19171 describes a process to obtain xylanases from *Thermotoga maritima, T. neapolitana* and *T. thermarum.*

DNA and proteins sequences of endo-xylanases from several strains and species of *Thermotoga* xylanases have been published. These xylanases may be grouped into two types by sequence homology with *T. maritima* XynA and XynB.

None of the references, at least none of the references disclosing a very high temperature optimum and almost no activity at 40°C, describes the use of an isolated *Thermotoga* xylanase, such as XynB, as a feed additive.

The xylanases may be obtained from different sources. Xylanases may be isolated/purified after having grown a selected *Thermotoga* strain in a suitable cultivation medium.

The xylanases may be obtained by cultivating a recombinant strain expressing a gene coding for the corresponding protein (e.g. XynB).

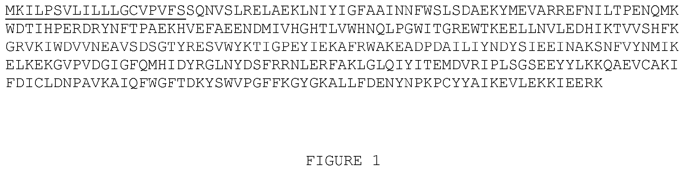

Suitable genes may chosen among those coding for the endo-1,4-beta-xylanase from *Thermotoga* sp. RQ2 (GenBank: ACB09229.1), the endo-1,4-beta-xylanase from *Thermotoga naphthophila* RKU-10 (GenBank: ADA66795.1), the xylanase from *Thermotoga neapolitana* (GenBank: CAA90235.1), the endo-1,4-beta-xylanase from *Thermotoga* sp. FjSS3-B.1 (GenBank: AAA90913.1) or the endo-1,4-beta-xylanase B from *Thermotoga maritima* MSB8 (GenBank: AAD35164.1 - figure 1 - SEQ ID NO 1).

Furthermore, variants of these enzymes that display an optimal temperature of activity (thermoactivity) and a thermostability that are in the ranges described above may be obtained by modifying the coding sequence of these genes.

Sequence modifications may include but are not limited to amino acid substitution(s), deletion(s) and/or insertion(s). Preferred sequence modifications are conservative substitutions (e.g. labelled as positive after a BLASTp analysis).

Preferably the variants enzymes have at least 80% amino acid sequence identity with *Thermotoga maritima* MSB8 XynB, more preferably at least 90%, even more preferably at least 95% or have at least 90% amino acid sequence similarity with *Thermotoga maritima* MSB8 XynB, more preferably at least 93%, 94%, 95% even more preferably at least 98%.

The recombinant xylanase may be expressed in any suitable host organism. Particularly suited hosts are bacteria, yeast and fungi. Preferred bacterial hosts are strains of *Escherichia coli* and *Bacillus* (*subtilis, licheniformis, amyloliquefaciens, megaterium*...) or *Streptomyces* (*coelicolor, lividans,* ...).

Preferred yeast hosts are strains of *Saccharomyces cerevisiae, Pichia pastoris* or *Yarrowia lipolytica.* Preferred fungal hosts are strains of *Aspergillus* (*nidulans, niger,...*), *Penicillium* and *Trichoderma.*

The xylanases may further be obtained under the form of a transgenic plant and/or seed expressing the corresponding enzyme or from a transgenic animal.

The xylanase may be prepared by cultivating the *Thermotoga* or the recombinant microbial strain in a suitable medium for growth and expression of the xylanase. Preferably the strains are cultivated in fermentors.

After cultivation, the xylanase may be recovered as a culture supernatant or a cell-extract. The supernatant or cell-extract is preferably further purified to obtain a semi-purified or a purified preparation. Suitable purification methods include but are not limited to centrifugation, microfiltration, ultrafiltration, precipitation, chromatography, ...

The xylanases may be provided as a liquid or a dry (powder) preparation. Liquid preparations are preferably stabilized by the addition of a suitable component such as salt (NaCl) or glycerol. Powder preparations may be obtained for instance by spray-drying or freeze-drying. The xylanases may be coated.

### Enzyme activity determination

Several methods are available to determine the enzymatic activity of endoxylanases in liquid preparations. Particularly suited is a method using the property of endoxylanases to hydrolyze xylan as substrate. The hydrolysis reaction liberates reducing sugars that cause a typical color to be developed by reaction with dinitrosalicylic acid (DNS). The color intensity at 570nm is directly proportional to the xylanase activity in the sample, provided that the substrate concentration remains sufficient (i.e. not more than 10% of degradation and/or a saturating concentration of the substrate is kept throughout the test).

One DXU of xylanase is defined as the amount of enzyme that liberates 1 pmole of reducing sugars (as xylose equivalent) per minute from a solution of 3% (w:v) birchwood xylan and in 100 mM citrate-phosphate buffer at pH 6 at 70°C. Reaction is performed for 15 minutes in a volume of 0.8 ml (0.7 ml substrate + 0.1 ml of diluted xylanase). After incubation, the reaction is terminated by addition of 1 ml of DNS reagent (composition : NaOH 400 mM, 3,5-dinitrosalicylic acid 40 mM, potassium sodium tartrate 1 M). The mixture is subsequently kept at 95°C for 15 min, and then cooled at 25°C for 5 min. Finally, the absorbance at 570 nm is measured against a control.

To assess the xylanase activity (DXU/ml) in terms of reducing sugar formation, a xylose standard curve is prepared with xylose instead of enzyme dilution preparation. All substrate incubations and subsequent measurements are performed in triplicate.

### Endoxylanase optimum activity temperature & thermostability determination

Temperature dependency of xylanase activity is analyzed with a variant of the above described dinitrosalicylic acid (DNS) reducing sugar method. The DNS method is performed on enzyme/substrate mixes incubated in an oil bath at temperatures ranging from 20 to 100°C. All other parameters of the method are kept unchanged.

Temperature stability is monitored by preincubating the enzyme preparations in a bath at temperatures ranging from 50 to 100°C. Samples are taken after 0, 10, 30, 60 (and 90) minutes and cooled in an ice bath for 10 min. Residual activity is assayed in triplicate with the method described above.

### Feed composition

The animal feeds (including silages) may comprise cereals such as wheat, barley, rye, maize, rice, sorghum, spelt, triticale or oats or cereal byproducts such as wheat bran, barley straw, maize cobs, oat millings, wheat middlings, wheat glutenfeed, rice germs or maize bran, or other plant materials such as soybeans or other legumes, rapeseed, lupine, peas, tapioca meal, beet molasses, potato pulps, peanut meal. Preferred cereals are wheat, barley and rye.

Preferably, the animal feed composition of the invention contains at least one vegetable protein or protein source. Examples of vegetable protein or protein sources are soybean, and cereals such as barley, maize, oat, rice, rye, sorghum and wheat.

The animal feed composition of the invention advantageously contains 0-80% maize; and/or 0-80% sorghum; and/or 0-70% wheat; and/or 0-70% barley; and/or 0-30% oats; and/or 0-40% soybean meal; and/or 0-10% fish meal; and/or 0-20% whey.

The animal feed composition of the invention advantageously contains additional feed ingredients or additives such as enzymes, prebiotics, probiotics, minerals and trace elements, vitamins and provitamins, vegetable, microbial or animal proteins, amino acids, their salts and analogs, starch, fibers, carbohydrates and sugars or sweeteners, oils and oil meals, vegetable or animals fats, dyes and other colourants, emulsifying and stabilizing agents, thickeners and gelling agents, binders, anti-caking agents and coagulants, preservatives, acidity regulators, carotenoids and xanthophylls, urea and its derivatives, digestibility enhancers, gut flora stabilizers, coccidiostats and other medical substances.

Preferred additional enzymes are selected from the group consisting of phytases, amylases, cellulases, glucanases, proteases, mannanases, pectinases, hemicellulases and (less preferably) (other) xylanases.

Preferred minerals are selected from the group consisting of salts (chlorides, sulfates, fluorides, carbonates, oxides,...), calcium, phosphorus, magnesium, potassium, sodium, manganese, zinc, nickel, molybdenium, copper, iron, selenium, cobalt and iodine.

Preferred vitamins are selected from the group consisting of vitamin A, carotene, ascorbic acid, vitamin D, D3, E, K, B1 thiamin, B6 pyridoxine, biotin, choline, folic acid, niacin, panthotenic acid, B2 riboflavin, cyanocobalamin,...

Suitable proteins are selected from the group consisting of vegetable, microbial or animal proteins.

Suitable amino acids are selected from the group consisting of methionine, cysteine, lysine, threonine, tryptophan, isoleucine, leucine, valine, histidine, arginine, glycine, serine, phenylalanine, tyrosine, aspartic acid, glutamic acid, proline.

Suitable micro-organisms belong but are not limited to *Aspergillus, Bacillus, Bifidobacterium, Enterococcus, Lactobacillus, Lactococcus, Leuconostoc, Pediococcus, Saccharomyces, Streptococcus.*

Possible other additives can be used, and found, for example, in the Community Register of Feed Additives pursuant to Regulation (EC) N°1831/2003 - Appendixes 3a&4 - Annex: List of additives, and its updates.

### Feed manufacturing

Animals diets can be manufactured as mash feed (non-pelleted) or pelleted feed. Typically, the milled feed-stuffs are mixed and sufficient amount of essential vitamins and minerals are added according to the specifications for the animal species in question.

The composition of the invention can be added in the form of a solid or liquid enzyme formulation, or in the form of a feed additive, such as a premix.

A solid composition is typically added before or during the mixing step; and a liquid composition is typically added after the pelleting step.

However, the hyper thermostable and hyper thermoactive liquid enzyme composition can also be added before the pelleting step.

The dosage of the xylanase of the invention in the feed can be optimized using trial-and-error methods as known in the art. Different xylanases may have different optimum dosage ranges.

### Brief description of the figures

Figure 1 shows the amino acid sequence of the xylanase XynB from Thermotoga maritima MSB8. The signal peptide is underlined.
Figure 2 shows Coomassie blue stained SDS-polyacrylamide gels of the endoxylanases recovered after the successive purification steps. figure 2a: XynAΔNC; figure 2b: XynB.
Figure 3 shows the effect of the temperature on the activity of the endoxylanases XynAΔNC and XynB. Maximum activity has been set at 100%.
Figure 4 shows the residual activity of the endoxylanases after preincubation at various temperatures.

### Examples

### Example 1: preparation of Thermotoga endoxylanases.

### Cloning of the catalytic domains of the xylanases XynA and XynB from Thermotoga maritima

Based on the published sequences of the genes of *Thermotoga* xynA (GenBank/GenPept™ accession Z46264) and xynB (GenBank/GenPept™ accession AAD35164) the entire xynB gene and the DNA fragment corresponding to the catalytic domain of xynA gene (xynAΔNC) were PCR-amplified from the genomic DNA from *Thermotoga maritima* MSB8 (DSM3109/ATCC43589) following classical protocols.

The PCR products were cloned into the pGEM-T Vector SystemI (Promega), using the procedure recommended by the supplier, and used to transform *E. coli* DH5α® ultracompetent cells. Blue-white selection allowed selection of white colonies carrying the PCR-fragment. Purified plasmid preparations (Nucleospin plasmid, Macherey-Nagel) were sequenced on an ALF DNA sequencer (Pharmacia Biotech). Sequencing of the inserted fragment was carried out using the universal primers T7 and RP as well as primers corresponding to internal DNA sequences. The sequences obtained were identical to the published sequences (GenBank/genPept™ accession number Z46264 for xynA and accession number AAD35164 for xynB).

The DNA fragment corresponding to the catalytic domain derivative of the modular XynA (XynAΔNC) and the complete xynB gene were subcloned into the pET 22b (+) cloning vector (Novagen) . The resulting recombinant plasmids were transformed in *E. coli* BL21 (DE3) cells (Stratagene).

### Cultivation of the recombinant strains and production of the xylanases XynAΔNC and XynB from Thermotoga maritima

15 ml of a 5 hours preculture (37°C) of the *E. coli* BL21 (DE3) cells carrying the xylanase genes were centrifuged at 10000 g for 1 minute and the pellet was resuspended in 900 ml Terrific broth (12g/l Bacto tryptone (Difco), 24g/l yeast extract (Difco), 4ml/l glycerol, 12.54g/l K₂HPO₄, 2.31g/l KH₂PO₄) containing 200µg/ml ampicillin in a 3 liter shake flask. The cultures were incubated at 37°C and 250 rpm until an absorbance at 550nm of between 3 & 4 was reached whereupon the expression of the enzyme was induced with 1mM isopropyl-1-thio-β-galactopyranoside.

After 15 hours incubation at 37°C the cells were harvested by centrifugation at 18000 g for 30 minutes at 4°C, resuspended in 50mM BICINE containing 10mM NaCl, disrupted in a prechilled cell disrupter (Constant Systems Ltd., Warwick, UK) at 28Kpsi and centrifuged at 40,000 g for 30 minutes. Chromosomal DNA was removed from the crude cell lysates by treatment with 0.2% protamine sulfate (Calbiochem) and centrifugation at 40,000 g for 30 minutes. 25 units of benzonase (Merck, Darmstadt, Germany) were then added to the solution.

### Recovery of the recombinant xylanases XynAΔNC and XynB

The crude enzymatic preparations of XynAΔNC and of XynB from *Thermotoga maritima* expressed in *Escherichia coli* have been concentrated by ultrafiltration by recirculating the liquid preparations on a UF polyethersulphone Biomax membrane (cut-off = 5 kDa) on a Proflux system (Millipore). The concentrated enzyme solutions were filtered on a sterile filtration system, including several steps with different cut-off and finally an absolute filter (cut-off = 0.22 µM) (Millipore).

The concentrated liquid enzymes were dried using a fluid bed drying ProCell LabSystem (Glatt) where it is sprayed on a carrier (wheat flour), giving a low-dusting granulated enzyme preparation.

### Purification of the recombinant xylanases XynAΔNC and XynB

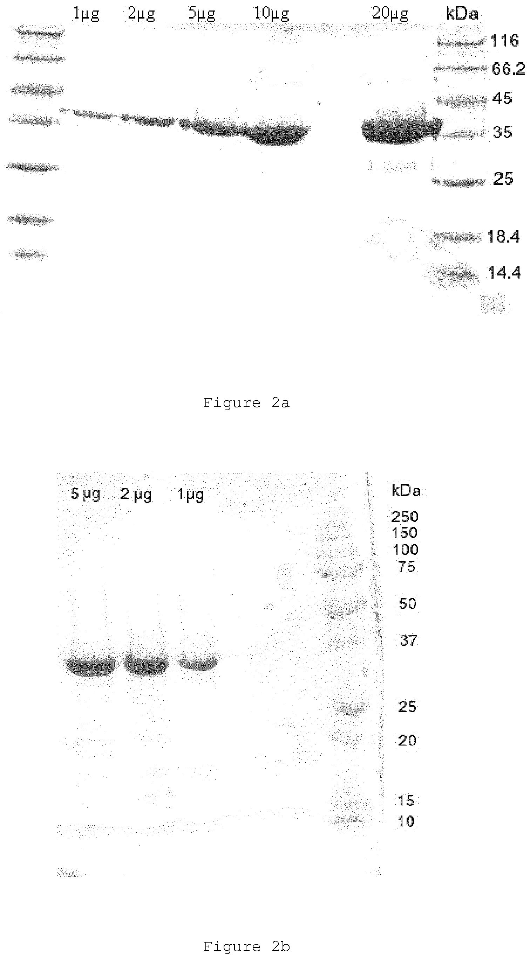

XynAΔNC was purified by using a three-step procedure. The crude extract was dialyzed 2500x against chromatographic buffer (Tris-HCl 20 mM pH 8.0) and was loaded on a Q HP XK 16/20 (GE Healthcare), a strong anion exchange column and eluted with a linear gradient (0 - 1 M NaCl). The semi-purified preparation was then dialyzed 2500x against chromatographic buffer (Tris-HCl 20 mM pH 8.0) and loaded on a Sephacryl S-100 HR 120 ml XK 16/70 (GE Healthcare), a size exclusion chromatography column. The active fractions were pooled, dialyzed 2500x against chromatographic buffer (Tris-HCl 20 mM pH 8.0) and loaded on a Mono-Q GL 5/50 (GE Healthcare), a strong anion exchange column and eluted with a linear gradient (0 - 1 M NaCl). The purified fractions have been pooled and their purity has been checked on SDS-PAGE (figure 2a).

XynB was purified by using a two-step procedure. The majority of the host proteins were removed by heat treatment of the enzymatic preparation (30 min at 75°C) followed by centrifugation (12000 x g at 4°C). The supernatant was dialyzed 2500x against chromatographic buffer (Bis-Tris 20 mM pH 6.2, loaded on a Q-Sepharose FF (GE Healthcare) ion exchange column and eluted with a linear gradient (0 - 1 M NaCl). The purity of recombinant xylanase has been checked on SDS-PAGE (figure 2b).

### Characterization of the recombinant enzymes

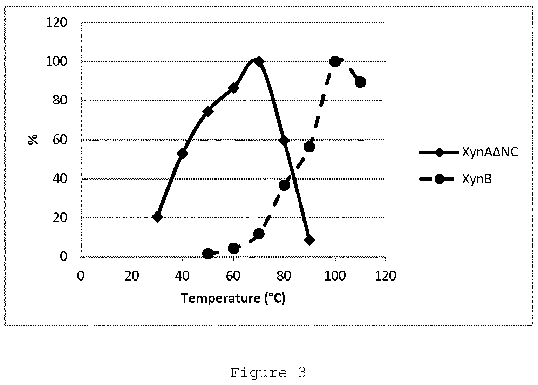

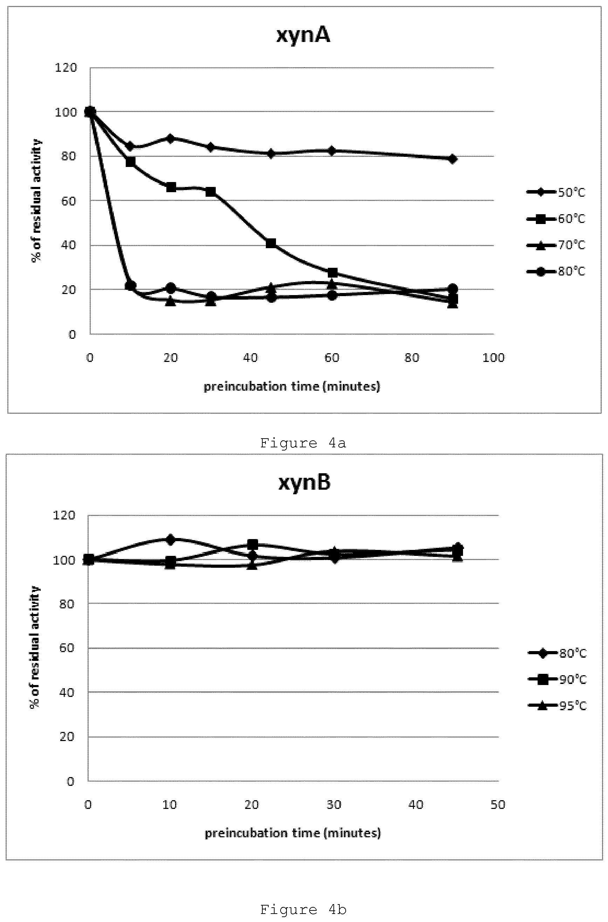

The xylanase activity of xynAΔNC and xynB was determined at various temperatures using the tests conditions as described. The maximum activities were 70°C and 90-95° respectively (figure 3).
The stability of the recombinant enzymes was evaluated using the protocol as described above. Results (figure 4) showed that XynAΔNC keeps 30% of its activity after incubation at 60°C for 60 min. XynB keeps 100% of its activity after an incubation at 90°C for 60 min.

### Example 2: Use of the Thermotoga maritima xylanases (XynAΔNC and XynB) in animal feed

A trial was performed using male or female broilers (Belgabroed). From 1 to day 42 of age they were kept in floor pens and offered a commercial broiler feed supplemented or not with exogenous xylanases. At the age of 1 day, the animals (768 birds - 384 males/384 females) were randomly distributed over 24 pens (4 treatments x 2 sexes x 3 replicates) of 32 birds/pen, 0.8m³/pen. At the age of 14 days, the animals were distributed in 48 pens (4 treatments x 2 sexes x 6 replicates) of 16 birds. Each pen is provided with 2 drinking nipples and a feeding tray. The temperature was 35°C at start, and then was decreased by 0.5°C per day; at 22 days, the temperature was maintained at 22°C. The light scheme was 23h30min light and 30min dark for the first period and 18h light and 6h dark for the second period. Birds were vaccinated against Newcastle disease at day 1 (at hatchery) and against Gumboro + Newcastle disease at day 14 (through drinking water).

Four treatments were included in this trial. To a wheat based diet (Table 1), either no enzyme was added (control), or a quantity equal to 500 DXU of *Thermotoga maritima* XynAΔNC/kg feed or a quantity equal to 400 DXU of *Thermotoga maritima* XynB/kg feed or a *Bacillus subtilis* xylanase (Belfeed™ B1100MP (E1606), an endo-1,4-beta-xylanase commercially available from Beldem - a division of Puratos, Groot-Bijgaarden, Belgium) supplemented at the recommended dosage of 10 UI/kg feed (positive control).

The inventors compared different levels of the positive control (the commercial xylanase Belfeed), such as 50 UI/kg feed and found that both parameters (BWG and FCR) reached a plateau at 10 UI/kg feed. The inventors therefore used this amount for the positive control.

The enzymes were added to the feed as liquids (XynAΔNC and XynB) or powder (Belfeed) incorporated into a premix prior to mixing it into the diet. The diets were offered ad libitum to the animals in the form of a mash. Water was also available freely.

Body weights were recorded at day 1, day 14 and day 42. Feed supply was recorded and the remaining feed in the tray was measured immediately after weighing 2 and 3.

Body weight gain (BWG) and feed conversion ratio (FCR) were determined for the periods 1-14 days, 14-42 days and 1-42 days of age.

**TABLE 1**

| Feed composition and contents of main nutrients. | |
|---|---|
| Raw materials | Content |
| Wheat | 57.20% |
| Soybean meal | 18.73% |
| Soybean heat treated | 13.00% |
| Animal fat | 4.00% |
| Wheat glutenfeed | 2.00% |
| Soybean oil | 0.467% |
| NaCl | 0.250% |
| Biolys | 0.283% |
| DL-Methionine | 0.233% |
| Threonine | 0.083% |
| CaC03 | 0.533% |
| Monocalcium Phosphate | 0.933% |
| Sodium bicarbonate | 0.100% |
| Vitamin/mineral premix | 1.500% |
| Natuphos premix | 0.500% |
| Betacid GM | 0.188% |
| | |
| Crude protein | 20.50% |
| Crude fat | 8.50% |
| Crude ashes | 6.00% |
| Crude fiber | 3.00% |
| Methionine | 0.53% |
| Phophorus tot. | 0.61% |
| | |
| Vitamin A | 15100 UI/kg |
| Vitamin D3 | 3000 UI/kg |
| Vitamin E | 30 mg/kg |
| Phytase (EC 3.1.3.8) E1600 | 500 FTU/kg |

The results of this trial are presented in Table 2 which shows the average BWG and FCR of the broilers in two periods.

**TABLE 2**

| | BWG (g/bird) | | | FCR (g/g) | | | Standard FCR |
|---|---|---|---|---|---|---|---|
| | 1-14 d | 14-42d | 1-42d | 1-14d | 14-42d | 1-42d | |
| Control | 315.0 | 1638.0 | 1961.4 | 1.394 | 1.819 | 1.691 | 1.737 |
| Belfeed | 330.4 | 1811.6 | 2146.2 | 1.368 | 1.728 | 1.626 | 1.587 |
| XynAΔNC | 327.6 | 1789.2 | 2129.4 | 1.368 | 1.775 | 1.659 | 1.667 |
| XynB | 319.2 | 1884.4 | 2205.0 | 1.361 | 1.720 | 1.624 | 1.562 |

Both growth and FCR improved for the diets containing XynAΔNC and XynB, both after 14 d. of starter period or after the total 42 d. fattening period. XynB showed a better performance than XynAΔNC.

### Example 3 : Use of the Thermotoga maritina xylanases (XynB) in animal feed

A trial was performed using male or female broilers (Belgabroed). From 1 to day 42 of age they were kept in floor pens and offered a commercial broiler feed supplemented or not with exogenous xylanases. At the age of 1 day, the animals (768 birds - 384 males/384 females) were randomly distributed over 24 pens (4 treatments x 2 sexes x 3 replicates) of 32 birds/pen, 0.8m³/pen. At the age of 14 days, the animals were distributed in 48 pens (4 treatments x 2 sexes x 6 replicates) of 16 birds. Each pen is provided with 2 drinking nipples and a feeding tray. The temperature was 35°C at start, and then was decreased by 0.5°C per day; at 22 days, the temperature was maintained at 22°C. The light scheme was 23h30min light and 30min dark for the first period and 18h light and 6h dark for the second period. Birds were vaccinated against Newcastle disease at day 1 (at hatchery) and against Gumboro + Newcastle disease at day 14 (through drinking water).

Four treatments were included in this trial. To a wheat based diet (Table 3), were added either a quantity equal to 200 DXU of XynB/kg feed or a quantity equal to 400 DXU of XynB/kg feed or a commercially available fungal xylanase Hostazym® X (Huvepharma; EC N°E1617) supplemented at the recommended dosage of 100 ppm or a *Bacillus subtilis* xylanase (Belfeed™ B1100MP (E1606), an endo-1,4-beta-xylanase commercially available from Beldem - a division of Puratos, Groot-Bijgaarden, Belgium) supplemented at the recommended dosage 10 UI/kg feed or 100 ppm.

The enzymes were added to the feed as powders incorporated into a premix prior to mixing it into the diet. The diets were offered ad libitum to the animals in the form of a mash. Water was also available freely.

Body weights were recorded at day 1, day 14 and day 42. Feed supply was recorded and the remaining feed in the trough was measured immediately after weighing 2 and 3.

Body weight gain (BWG) and feed conversion ratio (FCR) were determined for the periods 1-14 days, 14-42 days and 1-42 days of age.

**TABLE 3**

| Feed composition and contents of main nutrients. | |
|---|---|
| Raw materials | Content |
| Wheat | 58.19% |
| Soybean meal | 20.16% |
| Soybean heat treated | 10.00% |
| Animal fat | 4.00% |
| Rapeseed meal | 2.00% |
| Soybean oil | 1.133% |
| NaCl | 0.233% |
| Biolys | 0.283% |
| DL-Methionine | 0.233% |
| Threonine | 0.083% |
| CaCO3 | 0.500% |
| Monocalcium Phosphate | 0.968% |
| Sodium bicarbonate | 0.134% |
| Vitamin/mineral premix | 1.500% |
| Natuphos premix | 0.500% |
| Choline chloride | 0.083% |

The results of this trial are presented in Table 4 which shows the average BWG and FCR of the broilers in two periods.

**TABLE 4**

| | BWG (g/bird) | | | FCR (g/g) | | | Standard FCR |
|---|---|---|---|---|---|---|---|
| | 1-14d | 14-42d | 1-42d | 1-14 d | 14-42d | 1-42d | |
| Hostazyme | 308.0 | 2004.8 | 2318.4 | 1.492 | 1.787 | 1.714 | 1.604 |
| Belfeed | 315.0 | 2035.6 | 2339.4 | 1.462 | 1.727 | 1.660 | 1.537 |
| XynB 200u | 347.2 | 2088.8 | 2452.8 | 1.344 | 1.720 | 1.615 | 1.460 |
| XynB 400u | 334.6 | 2108.4 | 2469.6 | 1.342 | 1.708 | 1.611 | 1.454 |

Both growth and FCR significantly improved for the diets containing the XynB enzymes, both after 14 d. of starter period or after the total 42 d. fattening period. XynB showed a better BWG and FCR than other commercial xylanases.

## Claims

1. An animal feed being selected from the group consisting of silage, pelletized feed and mash feed, the said animal feed being supplemented with a composition comprising a xylanase, wherein the said xylanase is hyperthermophilic and hyperthermostable, wherein the optimum temperature of the xylanolytic activity present in the said composition is higher than 80°C, wherein more than 70% of the xylanolytic activity present in the said composition is resistant to 30 minutes of heating at 90°C and wherein the ratio of activity of the xylanolytic activity present in the said composition at optimal temperature and at 40°C is higher than 10.

2. The animal feed of claim 1, wherein the optimum temperature of the xylanolytic activity present in the said composition is higher than 85°C, preferably higher than 90°C.

3. The animal feed of claim 1 or 2, wherein the ratio of activity of the xylanolytic activity present in the said composition at optimal temperature and at 40°C is higher than 20.

4. The animal feed according to any of the preceding claims, wherein the hyperthermophilic and hyperthermostable xylanase has more than 80% identity and/or 95% of similarity with a xylanase selected from the group consisting of SEQ.ID.NO:1, SEQ.ID.NO:2, SEQ.ID.NO:3, SEQ.ID.NO:4, SEQ.ID.NO:5, amino acids 21-332 of SEQ.ID.NO:1, of SEQ.ID.NO:2, of SEQ.ID.NO:3, of SEQ.ID.NO:4 and of SEQ.ID.NO:5.

5. The animal feed according to any of the preceding claims wherein the hyperthermophilic and hyperthermostable xylanase has more than 99% identity with a xylanase selected from the group consisting of SEQ.ID.NO:1, SEQ.ID.NO:2, SEQ.ID.NO:3, SEQ.ID.NO:4, SEQ.ID.NO:5, amino acids 21-332 of SEQ.ID.NO:1, of SEQ.ID.NO:2, of SEQ.ID.NO:3, of SEQ.ID.NO:4 and of SEQ.ID.NO:5.

6. The animal feed according to any of the preceding claims comprising between 0.08 and 40 mg/kg of the said hyperthermophilic and hyperthermostable xylanase.

7. The animal feed according to any of the preceding claims comprising at least 50% (on dry weight) of plant material.

8. The animal feed of claim 7, wherein the plant material is selected from the group consisting of cereals, legumes, beet molasse, potato pulps and peanut meal.

9. The animal feed according to any of the preceding claims comprising at least 5% (dry weight) proteins and/or at least 2% (dry weight) fat.

10. Use of a hyperthermophilic and hyperthermostable xylanase for improving the body weight gain and/or the feed conversion ratio in an animal, wherein the said hyperthermophilic and hyperthermostable xylanase has a temperature optimum higher than 80°C, wherein more than 70% of the xylanolytic activity of the said hyperthermophilic and hyperthermostable xylanase is resistant to 30 minutes of heating at 90°C and wherein the ratio of activity at optimal temperature and at 40°C of the said hyperthermophilic and hyperthermostable xylanase is higher than 10, wherein the use is a non-therapeutical use.

11. Use of the animal feed according to any of the preceding claims 1 to 9 in improving the body weight gain and/or the feed conversion ratio in an animal, wherein the use is a non-therapeutical use.

12. The use of a hyperthermophilic and hyperthermostable xylanase or an animal feed according to claim 10 or 11, wherein the animal is a non-ruminant vertebrate, or a crustacean and is preferably a fish, a pig or poultry, more preferably pig or poultry.

13. A method to produce the animal feed according to any of the preceding claims 1 to 9 comprising the steps of
a) selecting a feed comprising hemicellulose;
b) adding a composition comprising hyperthermophilic and hyperthermostable xylanase, wherein more than 70% of the xylanolytic activity present in the said composition is resistant to 30 minutes of heating the said composition at 90°C, wherein the optimum temperature of the xylanolytic activity present in the said composition is higher than 80°C, and wherein the ratio of activity of the xylanolytic activity present in the said composition at optimal temperature and at 40°C is higher than 10.

14. The method of claim 13, wherein the xylanase activity present in the said composition is measured using 3% (w:v) xylan, preferably using 3% birchwood xylan, as substrate.

15. The method of claim 13 or 14, wherein the xylanase activity present in the said composition is measured after a 15-minute reaction.

## Patentansprüche

1. Tierfutter, das ausgewählt ist aus der Gruppe bestehend aus Silofutter, pelletiertem Futter und Maische Futter, wobei das Tierfutter mit einer Zusammensetzung ergänzt ist, die eine Xylanase umfasst, wobei die Xylanase hyperthermophil und hyperthermostabil ist, wobei die optimale Temperatur der in der Zusammensetzung vorhandenen xylanolytischen Aktivität höher als 80°C ist, wobei mehr als 70% der in der Zusammensetzung vorhandenen xylanolytischen Aktivität gegenüber 30 Minuten Erhitzen auf 90°C beständig sind und wobei das Aktivitätsverhältnis der in der Zusammensetzung vorhandenen xylanolytischen Aktivität bei optimaler Temperatur und bei 40°C höher als 10 ist.

2. Tierfutter nach Anspruch 1, wobei die optimale Temperatur der in der Zusammensetzung vorhandenen xylanolytischen Aktivität höher als 85°C, vorzugsweise höher als 90°C, ist.

3. Tierfutter nach Anspruch 1 oder 2, wobei das Aktivitätsverhältnis der in der Zusammensetzung vorhandenen xylanolytischen Aktivität bei optimaler Temperatur und bei 40°C höher als 20 ist.

4. Tierfutter nach einem der vorhergehenden Ansprüche, wobei die hyperthermophile und hyperthermostabile Xylanase mehr als 80% Identität und/oder 95% Ähnlichkeit mit einer Xylanase aufweist, die ausgewählt ist aus der Gruppe bestehend aus SEQ.ID.NO:1, SEQ.ID.NO:2, SEQ.ID.NO:3, SEQ.ID.NO:4, SEQ.ID.NO:5, den Aminosäuren 21-332 von SEQ.ID.NO:1, von SEQ.ID.NO:2, von SEQ.ID.NO:3, von SEQ.ID.NO:4 und von SEQ.ID.NO:5.

5. Tierfutter nach einem der vorhergehenden Ansprüche, wobei die hyperthermophile und hyperthermostabile Xylanase eine Identität von mehr als 99% mit einer Xylanase aufweist, die ausgewählt ist aus der Gruppe bestehend aus SEQ.ID.NO:1, SEQ.ID.NO:2, SEQ.ID.NO:3, SEQ.ID.NO:4, SEQ.ID.NO:5, den Aminosäuren 21-332 von SEQ.ID.NO:1, von SEQ.ID.NO:2, von SEQ.ID.NO:3 von SEQ.ID.NO:4 und von SEQ.ID.NO:5.

6. Tierfutter nach einem der vorhergehenden Ansprüche, umfassend zwischen 0,08 und 40 mg/kg der hyperthermophilen und hyperthermostabilen Xylanase.

7. Tierfutter nach einem der vorhergehenden Ansprüche, umfassend mindestens 50% (bezogen auf das Trockengewicht) Pflanzenmaterial.

8. Tierfutter nach Anspruch 7, wobei das Pflanzenmaterial ausgewählt ist aus der Gruppe, bestehend aus Getreide, Hülsenfrüchten, Rübenmelasse, Kartoffelpulpe und Erdnussmehl.

9. Tierfutter nach einem der vorhergehenden Ansprüche, umfassend mindestens 5% (Trockengewicht) Proteine und/oder mindestens 2% (Trockengewicht) Fett.

10. Verwendung einer hyperthermophilen und hyperthermostabilen Xylanase zur Verbesserung der Körpergewichtszunahme und/oder des Futterumwandlungsverhältnisses bei einem Tier, wobei die hyperthermophile und hyperthermostabile Xylanase ein Temperaturoptimum von mehr als 80°C aufweist, wobei mehr als 70% der xylanolytischen Aktivität der hyperthermophilen und hyperthermostabilen Xylanase resistent gegen 30 Minuten Erhitzen auf 90°C ist und wobei das Aktivitätsverhältnis der hyperthermophilen und hyperthermostabilen Xylanase bei optimaler Temperatur und bei 40°C höher als 10 ist, wobei die Verwendung eine nicht-therapeutische Verwendung ist.

11. Verwendung des Tierfutters nach einem der vorhergehenden Ansprüche 1 bis 9 zur Verbesserung der Körpergewichtszunahme und/oder des Futterumwandlungsverhältnisses bei einem Tier, wobei die Verwendung eine nicht-therapeutische Verwendung ist.

12. Verwendung einer hyperthermophilen und hyperthermostabilen Xylanase oder eines Tierfutters nach Anspruch 10 oder 11, wobei das Tier ein Nichtwiederkäuer-Wirbeltier oder ein Krustentier ist und vorzugsweise ein Fisch, ein Schwein oder Geflügel, stärker bevorzugt Schwein oder Geflügel, ist.

13. Verfahren zur Herstellung des Tierfutters nach einem der vorhergehenden Ansprüche 1 bis 9, umfassend die Schritte
a) Auswählen eines Futtermittels, umfassend Hemicellulose;
b) Zugeben einer Zusammensetzung, umfassend hyperthermophile und hyperthermostabile Xylanase, wobei mehr als 70% der in der Zusammensetzung vorhandenen xylanolytischen Aktivität gegenüber 30 Minuten Erhitzen der Zusammensetzung auf 90°C beständig ist, wobei die optimale Temperatur der in der Zusammensetzung vorhandenen xylanolytischen Aktivität höher als 80°C ist und wobei das Aktivitätsverhältnis der in der Zusammensetzung vorhandenen xylanolytischen Aktivität bei optimaler Temperatur und bei 40°C höher als 10 ist.

14. Verfahren nach Anspruch 13, wobei die in der Zusammensetzung vorhandene Xylanaseaktivität unter Verwendung von 3% (Gew./Vol.) Xylan, vorzugsweise unter Verwendung von 3% Birkenholz-Xylan, als Substrat gemessen wird.

15. Verfahren nach Anspruch 13 oder 14, wobei die in der Zusammensetzung vorhandene Xylanaseaktivität nach einer 15-minütigen Reaktion gemessen wird.

## Revendications

1. Aliment pour animaux, choisi dans le groupe constitué par l'ensilage, les aliments en pellets et les aliments sous forme de farine, ledit aliment pour animaux étant complémenté par une composition comprenant une xylanase, où ladite xylanase est hyper thermophile et hyper thermostable, où la température optimale de l'activité xylanolytique présente dans ladite composition est supérieure à 80 °C, où plus de 70 % de l'activité xylanolytique présente dans ladite composition est résistante à 30 minutes de chauffage à 90 °C, et où le rapport de l'activité xylanolytique présente dans ladite composition à une température optimale et à 40 °C est supérieur à 10.

2. Aliment pour animaux selon la revendication 1, dans lequel la température optimale de l'activité xylanolytique présente dans ladite composition est supérieure à 85 °C, préférablement supérieure à 90 °C.

3. Aliment pour animaux selon la revendication 1 ou 2, dans lequel le rapport de l'activité xylanolytique présente dans ladite composition à une température optimale et à 40 °C est supérieur à 20.

4. Aliment pour animaux selon l'une quelconque des revendications précédentes, dans lequel la xylanase hyper thermophile et hyper thermostable possède plus de 80 % d'identité et/ou 95 % de similarité avec une xylanase choisie dans le groupe constitué par SEQ ID n° 1, SEQ ID n° 2, SEQ ID n° 3, SEQ ID n° 4, SEQ ID n° 5, les acides aminés 21-332 de SEQ ID n° 1, de SEQ ID n° 2, de SEQ ID n° 3, de SEQ ID n° 4 et de SEQ ID n° 5.

5. Aliment pour animaux selon l'une quelconque des revendications précédentes, dans lequel la xylanase hyper thermophile et hyper thermostable possède plus de 99 % d'identité avec une xylanase choisie dans le groupe constitué par SEQ ID n° 1, SEQ ID n° 2, SEQ ID n° 3, SEQ ID n° 4, SEQ ID n° 5, les acides aminés 21-332 de SEQ ID n° 1, de SEQ ID n° 2, de SEQ ID n° 3, de SEQ ID n° 4 et de SEQ ID n° 5.

6. Aliment pour animaux selon l'une quelconque des revendications précédentes, comprenant entre 0,08 et 40 mg/kg de ladite xylanase hyper thermophile et hyper thermostable.

7. Aliment pour animaux selon l'une quelconque des revendications précédentes, comprenant au moins 50 % (en poids sec) de matériau végétal.

8. Aliment pour animaux selon la revendication 7, dans lequel le matériau végétal est choisi dans le groupe constitué par les céréales, les légumineuses, la mélasse de betterave, les pulpes de pomme de terre et la farine d'arachide.

9. Aliment pour animaux selon l'une quelconque des revendications précédentes, comprenant au moins 5 % (en poids sec) de protéines et/ou au moins 2 % (en poids sec) de matières grasses.

10. Utilisation d'une xylanase hyper thermophile et hyper thermostable pour l'amélioration du gain de poids corporel et/ou du taux de conversion alimentaire chez un animal, dans laquelle ladite xylanase hyper thermophile et hyper thermostable possède un optimum de température supérieur à 80 °C, où plus de 70 % de l'activité xylanolytique de ladite xylanase hyper thermophile et hyper thermostable est résistante à 30 minutes de chauffage à 90 °C, et où le rapport de l'activité à la température optimale et à 40 °C de ladite xylanase hyper thermophile et hyper thermostable est supérieur à 10, où l'utilisation est une utilisation non thérapeutique.

11. Utilisation de l'aliment pour animaux selon l'une quelconque des revendications 1 à 9 précédentes, pour l'amélioration du gain de poids corporel et/ou du taux de conversion alimentaire chez un animal, où l'utilisation est une utilisation non thérapeutique.

12. Utilisation d'une xylanase hyper thermophile et hyper thermostable ou d'un aliment pour animaux selon la revendication 10 ou 11, dans laquelle l'animal est un vertébré non ruminant, ou un crustacé, et est de préférence un poisson, un porc, ou une volaille, plus préférablement un porc ou une volaille.

13. Méthode de production de l'aliment pour animaux selon l'une quelconque des revendications 1 à 9 précédentes, comprenant les étapes consistant à
a) sélectionner un aliment comprenant de l'hémicellulose ;
b) ajouter une composition comprenant une xylanase hyper thermophile et hyper thermostable, où plus de 70 % de l'activité xylanolytique présente dans ladite composition est résistante à 30 minutes de chauffage de ladite composition à 90 °C, où la température optimale de l'activité xylanolytique présente dans ladite composition est supérieure à 80 °C, et où le rapport de l'activité de l'activité xylanolytique présente dans ladite composition à une température optimale et à 40 °C est supérieur à 10.

14. Méthode selon la revendication 13, dans laquelle l'activité de xylanase présente dans ladite composition est mesurée en utilisant du xylane à 3 % (p/v), de préférence en utilisant 3 % de xylane de bois de bouleau, comme substrat.

15. Méthode selon la revendication 13 ou 14, dans laquelle l'activité de xylanase présente dans ladite composition est mesurée après une réaction de 15 minutes.


### SEQUENCE LISTING

<110> Puratos NV
<120> A feed composition supplemented with a xylanase
<130> BPPura0052PC00
<160> 5
<170> PatentIn version 3.3
<210> 1
   <211> 347
   <212> PRT
   <213> Thermotoga maritima
<220>
   <221> MISC_FEATURE
   <222> (1)..(347)
   <223> Strain MSB8; GeneBank AAD35164.1
<220>
   <221> SIGNAL
   <222> (1)..(20)
   <223> Predicted signal peptide
<400> 1
   Met Lys Ile Leu Pro Ser Val Leu Ile Leu Leu Leu Gly Cys Val Pro
   1               5                   10                  15
   Val Phe Ser Ser Gln Asn Val Ser Leu Arg Glu Leu Ala Glu Lys Leu
               20                  25                  30
   Asn Ile Tyr Ile Gly Phe Ala Ala Ile Asn Asn Phe Trp Ser Leu Ser
           35                  40                  45
   Asp Ala Glu Lys Tyr Met Glu Val Ala Arg Arg Glu Phe Asn Ile Leu
       50                  55                  60
   Thr Pro Glu Asn Gln Met Lys Trp Asp Thr Ile His Pro Glu Arg Asp
   65                  70                  75                  80
   Arg Tyr Asn Phe Thr Pro Ala Glu Lys His Val Glu Phe Ala Glu Glu
                   85                  90                  95
   Asn Asp Met Ile Val His Gly His Thr Leu Val Trp His Asn Gln Leu
               100                 105                 110
   Pro Gly Trp Ile Thr Gly Arg Glu Trp Thr Lys Glu Glu Leu Leu Asn
           115                 120                 125
   Val Leu Glu Asp His Ile Lys Thr Val Val Ser His Phe Lys Gly Arg
           130                 135                 140
   Val Lys Ile Trp Asp Val Val Asn Glu Ala Val Ser Asp Ser Gly Thr
   145                 150                 155                 160
   Tyr Arg Glu Ser Val Trp Tyr Lys Thr Ile Gly Pro Glu Tyr Ile Glu
                   165                 170                 175
   Lys Ala Phe Arg Trp Ala Lys Glu Ala Asp Pro Asp Ala Ile Leu Ile
               180                 185                 190
   Tyr Asn Asp Tyr Ser Ile Glu Glu Ile Asn Ala Lys Ser Asn Phe Val
           195                 200                 205
   Tyr Asn Met Ile Lys Glu Leu Lys Glu Lys Gly Val Pro Val Asp Gly
       210                 215                 220
   Ile Gly Phe Gln Met His Ile Asp Tyr Arg Gly Leu Asn Tyr Asp Ser
   225                 230                 235                 240
   Phe Arg Arg Asn Leu Glu Arg Phe Ala Lys Leu Gly Leu Gln Ile Tyr
                   245                 250                 255
   Ile Thr Glu Met Asp Val Arg Ile Pro Leu Ser Gly Ser Glu Glu Tyr
               260                 265                 270
   Tyr Leu Lys Lys Gln Ala Glu Val Cys Ala Lys Ile Phe Asp Ile Cys
           275                 280                 285
   Leu Asp Asn Pro Ala Val Lys Ala Ile Gln Phe Trp Gly Phe Thr Asp
       290                 295                 300
   Lys Tyr Ser Trp Val Pro Gly Phe Phe Lys Gly Tyr Gly Lys Ala Leu
   305                 310                 315                 320
   Leu Phe Asp Glu Asn Tyr Asn Pro Lys Pro Cys Tyr Tyr Ala Ile Lys
                   325                 330                 335
   Glu Val Leu Glu Lys Lys Ile Glu Glu Arg Lys
               340                 345
<210> 2
   <211> 347
   <212> PRT
   <213> Thermotoga sp.
<220>
   <221> MISC_FEATURE
   <223> Strain RQ2; GeneBank ACB09229.1
<220>
   <221> SIGNAL
   <222> (1)..(20)
   <223> Predicted signal peptide
<400> 2
   Met Lys Ile Leu Pro Ser Val Leu Ile Leu Leu Leu Gly Cys Val Pro
   1               5                   10                  15
   Val Phe Ser Ser Gln Asn Val Ser Leu Arg Glu Leu Ala Glu Lys Leu
               20                  25                  30
   Asn Ile Tyr Val Gly Phe Ala Ala Ile Asn Asn Phe Trp Ser Leu Ser
           35                  40                  45
   Asp Ala Glu Lys Tyr Met Glu Val Ala Arg Arg Glu Phe Asn Ile Leu
       50                  55                  60
   Thr Pro Glu Asn Gln Met Lys Trp Asp Thr Ile His Pro Glu Arg Asp
   65                  70                  75                  80
   Arg Tyr Asn Phe Thr Pro Ala Glu Lys His Val Glu Phe Ala Glu Glu
                   85                  90                  95
   Asn Asn Met Ile Val His Gly His Thr Leu Val Trp His Asn Gln Leu
               100                 105                 110
   Pro Gly Trp Ile Thr Gly Arg Glu Trp Thr Lys Glu Glu Leu Leu Asn
           115                 120                 125
   Val Leu Glu Asp His Ile Lys Thr Val Val Ser His Phe Lys Gly Arg
       130                 135                 140
   Val Lys Ile Trp Asp Val Val Asn Glu Ala Val Ser Asp Ser Gly Thr
   145                 150                 155                 160
   Tyr Arg Glu Ser Ile Trp Tyr Lys Thr Ile Gly Pro Glu Tyr Ile Glu
                   165                 170                 175
   Lys Ala Phe Arg Trp Ala Lys Glu Ala Asp Pro Asp Ala Ile Leu Ile
               180                 185                 190
   Tyr Asn Asp Tyr Ser Ile Glu Glu Ile Asn Ala Lys Ser Asn Phe Val
           195                 200                 205
   Tyr Asn Met Ile Lys Glu Leu Lys Glu Lys Gly Val Pro Val Asp Gly
       210                 215                 220
   Ile Gly Phe Gln Met His Ile Asp Tyr Arg Gly Leu Asn Tyr Asp Ser
   225                 230                 235                 240
   Phe Arg Arg Asn Leu Glu Arg Phe Ala Lys Leu Gly Leu Gln Ile Tyr
                   245                 250                 255
   Ile Thr Glu Met Asp Val Arg Ile Pro Leu Ser Gly Ser Glu Glu Tyr
               260                 265                 270
   Tyr Leu Lys Lys Gln Ala Glu Val Cys Ala Lys Ile Phe Asp Ile Cys
           275                 280                 285
   Leu Asp Asn Pro Ala Val Lys Ala Ile Gln Phe Trp Gly Phe Thr Asp
       290                 295                 300
   Lys Tyr Ser Trp Val Pro Gly Phe Phe Lys Gly Tyr Gly Lys Ala Leu
   305                 310                 315                 320
   Leu Phe Asp Glu Asn Tyr Asn Pro Lys Pro Cys Tyr Tyr Ala Ile Lys
                   325                 330                 335
   Glu Val Leu Glu Lys Lys Ile Glu Glu Arg Lys
               340                 345
<210> 3
   <211> 344
   <212> PRT
   <213> Thermotoga naphtophila
<220>
   <221> MISC_FEATURE
   <222> (1)..(344)
   <223> Strain RKU-10; GeneBank ADA66795.1
<220>
   <221> SIGNAL
   <222> (1)..(20)
   <223> Predicted signal peptide
<400> 3
   Met Lys Ile Leu Pro Ser Val Leu Ile Leu Leu Leu Gly Cys Val Pro
   1               5                   10                  15
   Val Phe Ser Ser Gln Asn Val Ser Leu Arg Glu Leu Ala Glu Lys Leu
               20                  25                  30
   Asn Ile Tyr Ile Gly Phe Ala Ala Val Asn Asn Phe Trp Ser Leu Pro
           35                  40                  45
   Asp Ala Glu Lys Tyr Met Glu Ile Ala Arg Arg Glu Phe Asn Ile Leu
       50                  55                  60
   Thr His Glu Asn Gln Met Lys Trp Asp Thr Ile His Pro Glu Arg Asp
   65                  70                  75                  80
   Arg Tyr Asn Phe Thr Pro Ala Glu Lys His Val Glu Phe Ala Glu Glu
                   85                  90                  95
   Asn Gly Met Ile Val His Gly His Thr Leu Val Trp His Asn Gln Leu
               100                 105                 110
   Pro Gly Trp Leu Thr Gly Arg Glu Trp Thr Arg Glu Glu Leu Leu Asn
           115                 120                 125
   Val Leu Glu Asp His Ile Lys Thr Val Val Ser His Phe Lys Gly Arg
       130                 135                 140
   Val Lys Ile Trp Asp Val Val Asn Glu Ala Val Ser Asp Ser Gly Thr
   145                 150                 155                 160
   Tyr Arg Glu Ser Val Trp Tyr Lys Thr Ile Gly Pro Glu Tyr Ile Glu
                   165                 170                 175
   Lys Ala Phe Arg Trp Ala Lys Glu Ala Asp Pro Asp Ala Ile Leu Ile
               180                 185                 190
   Tyr Asn Asp Tyr Ser Ile Glu Glu Ile Asn Ala Lys Ser Asn Phe Val
           195                 200                 205
   Tyr Asn Met Ile Lys Glu Leu Lys Glu Lys Gly Val Pro Val Asp Gly
       210                 215                 220
   Ile Gly Phe Gln Met His Ile Asp His Arg Gly Leu Asn Tyr Asp Ser
   225                 230                 235                 240
   Phe Arg Arg Asn Leu Glu Arg Phe Ala Glu Leu Gly Leu Gln Ile Tyr
                   245                 250                 255
   Ile Thr Glu Met Asp Val Arg Ile Pro Leu Ser Gly Ser Glu Glu Tyr
               260                 265                 270
   Tyr Leu Lys Lys Gln Ala Glu Val Cys Ala Lys Ile Phe Glu Ile Cys
           275                 280                 285
   Leu Lys Asn Pro Ala Val Lys Ala Ile Gln Phe Trp Gly Phe Thr Asp
       290                 295                 300
   Lys Tyr Ser Trp Val Pro Gly Phe Phe Lys Gly Tyr Gly Lys Ala Leu
   305 310 315 320
   Leu Phe Asp Glu Asn Tyr Asn Pro Lys Pro Cys Tyr Tyr Ala Ile Lys
   325 330 335
   Glu Val Leu Glu Lys Lys Arg Lys
   340
<210> 4
   <211> 346
   <212> PRT
   <213> Thermotoga neapolitana
<220>
   <221> MISC_FEATURE
   <222> (1)..(346)
   <223> GeneBank CAA90235.1
<220>
   <221> SIGNAL
   <222> (1)..(20)
   <223> Predicted signal peptide
<400> 4
   Met Lys Gly Leu Pro Ala Leu Leu Leu Leu Leu Ile Gly Cys Val Ser
   1 5 10 15
   Ser Phe Gly Ser Gln Asp Val Pro Leu Arg Val Leu Ala Glu Lys Leu
   20 25 30
   Asn Ile His Ile Gly Phe Ala Ala Gly Asn Asn Phe Trp Ser Leu Pro
   35 40 45
   Asp Ala Glu Lys Tyr Met Glu Val Ala Lys Arg Glu Phe Asn Ile Leu
   50 55 60
   Thr Pro Gly Asn Gln Met Lys Trp Asp Thr Ile His Pro Glu Arg Asn
   65 70 75 80
   Arg Tyr Asn Phe Glu Pro Ala Glu Lys His Val Glu Phe Ala Leu Lys
   85 90 95
   Asn Asp Met Ile Val His Gly His Thr Leu Val Trp His Asn Gln Leu
   100 105 110
   Pro Gly Trp Leu Thr Gly Gln Glu Trp Ser Lys Glu Glu Leu Leu Asn
   115 120 125
   Ile Leu Glu Asp His Val Lys Thr Val Val Ser His Phe Arg Gly Arg
       130                 135                 140
   Val Lys Ile Trp Asp Val Val Asn Glu Ala Val Ser Asp Ser Gly Thr
   145                 150                 155                 160
   Tyr Arg Glu Ser Ile Trp Tyr Arg Thr Ile Gly Pro Glu Tyr Ile Glu
                   165                 170                 175
   Lys Ala Leu Ile Trp Ala Lys Glu Ala Asp Pro Asp Ala Ile Leu Ile
               180                 185                 190
   Tyr Asn Asp Tyr Asn Ile Glu Glu Ile Asn Ala Lys Ser Asn Phe Val
           195                 200                 205
   Tyr Asn Met Ile Lys Asn Leu Arg Glu Lys Gly Val Pro Ile Asp Gly
       210                 215                 220
   Ile Gly Phe Gln Met His Ile Asp Tyr Arg Gly Ile Asn Tyr Glu Ser
   225                 230                 235                 240
   Phe Lys Lys Asn Leu Glu Arg Phe Ala Glu Leu Gly Leu Gln Ile Tyr
                   245                 250                 255
   Ile Thr Glu Met Asp Arg Gly Phe Pro Leu Gly Gly Ser Val Gly Tyr
               260                 265                 270
   Tyr Leu Lys Lys Gln Ala Glu Val Tyr Arg Arg Ile Phe Glu Ile Cys
           275                 280                 285
   Leu Asp Asn Pro Ala Val Arg Ala Ile Gln Phe Trp Gly Phe Thr Asp
       290                 295                 300
   Lys Tyr Ser Trp Val Pro Gly Phe Phe Lys Gly Tyr Gly Lys Ala Leu
   305                 310                 315                 320
   Ile Phe Asp Glu Asn Tyr Asn Pro Lys Pro Cys Tyr Phe Ala Ile Arg
                   325                 330                 335
   Glu Leu Met Glu Glu Lys Leu Lys Glu Arg
               340                 345
<210> 5
   <211> 346
   <212> PRT
   <213> Thermotoga sp. strain FjSS3-B.1
<220>
   <221> MISC_FEATURE
   <222> (1)..(346)
   <223> GeneBank AAA90913.1
<220>
   <221> SIGNAL
   <222> (1)..(20)
   <223> Predicted signal peptide
<400> 5
   Met Lys Gly Leu Pro Ala Leu Leu Leu Leu Leu Ile Gly Cys Val Ser
   1               5                   10                  15
   Ser Phe Gly Ser Gln Asp Val Pro Leu Arg Val Leu Ala Glu Lys Leu
               20                  25                  30
   Asn Ile His Ile Gly Phe Ala Ala Gly Asn Asn Phe Trp Ser Leu Pro
           35                  40                  45
   Asp Ala Glu Lys Tyr Met Glu Val Ala Lys Arg Glu Phe Asn Ile Leu
       50                  55                  60
   Thr Pro Glu Asn Gln Met Lys Trp Asp Thr Ile His Pro Glu Arg Asn
   65                  70                  75                  80
   Arg Tyr Asn Phe Glu Pro Ala Glu Lys His Val Glu Phe Ala Leu Lys
                   85                  90                  95
   Asn Asp Met Ile Val His Gly His Thr Leu Val Trp His Asn Gln Leu
               100                 105                 110
   Pro Gly Trp Leu Thr Gly Gln Glu Trp Ser Lys Glu Glu Leu Leu Asn
           115                 120                 125
   Ile Leu Glu Asp His Val Lys Thr Val Val Ser His Phe Arg Gly Arg
       130                 135                 140
   Val Lys Ile Trp Asp Val Val Asn Glu Ala Val Ser Asp Ser Gly Thr
   145                 150                 155                 160
   Tyr Arg Glu Ser Ile Trp Tyr Lys Thr Ile Gly Pro Glu Tyr Ile Glu
                   165                 170                 175
   Lys Ala Phe Ile Trp Ala Arg Glu Ala Asp Pro Asp Ala Val Leu Ile
               180                 185                 190
   Tyr Asn Asp Tyr Ser Ile Glu Glu Ile Asn Ala Lys Ser Asn Phe Val
           195                 200                 205
   Tyr Asn Met Ile Lys Asn Leu Lys Glu Lys Gly Val Pro Ile Asp Gly
       210                 215                 220
   Ile Gly Phe Gln Met His Ile Asp Tyr Arg Gly Leu Asn Tyr Glu Ser
   225                 230                 235                 240
   Phe Lys Lys Asn Leu Glu Arg Phe Ala Glu Leu Gly Leu Gln Ile Tyr
                   245                 250                 255
   Ile Thr Glu Met Asp Val Arg Ile Pro Leu Gly Gly Ser Glu Glu Tyr
               260                 265                 270
   Tyr Leu Lys Lys Gln Ala Glu Val Tyr Arg Arg Ile Phe Glu Ile Cys
           275                 280                 285
   Leu Asp Asn Pro Ala Val Arg Ala Ile Gln Phe Trp Gly Phe Thr Asp
       290                 295                 300
   Lys Tyr Ser Trp Val Pro Gly Phe Phe Lys Gly Tyr Gly Lys Ala Leu
   305                 310                 315                 320
   Ile Phe Asp Glu Asn Tyr Asn Pro Lys Pro Cys Tyr Phe Ala Ile Arg
                   325                 330                 335
   Glu Leu Met Glu Glu Lys Leu Lys Glu Arg
               340                 345